# FASCICULE DE BREVET EUROPEEN

(11) **EP 3 908 336 B1**
(45) Date de publication et mention de la délivrance du brevet: **23.08.2023**
(21) Numéro de dépôt: 20797570.7
(22) Date de dépôt: 19.10.2020
(51) Int. Cl.: A61L 9/14, A61L 9/12, A61L 9/03

(54) **SYSTEMES DE DIFFUSION INTELLIGENTS PAR NEBULISATION**
INTELLIGENTE SYSTEME ZUR AUSGABE DURCH VERNEBELUNG
INTELLIGENT SYSTEMS FOR DISPENSING BY NEBULISATION

(30) Priorité: 22.10.2019 FR 1911777
(43) Date de publication de la demande: 17.11.2021
(73) Titulaire: Chakroun, Ilyes, La Soukra Ariana, 2036 (TN)
(72) Inventeur: Chakroun, Ilyes, La Soukra Ariana, 2036 (TN)
(74) Mandataire: Nasr, Abdelmottaleb
(86) Numéro de dépôt international: PCT/IB2020/059799
(87) Numéro de publication internationale: WO 2021/079248

(56) Documents cités:
- WO-A1-2015/145452
- US-A1- 2019 061 466
- US-A1- 2019 085 852

## Description

La présente invention a trait au domaine technique des systèmes de diffusion, notamment par nébulisation, d'un liquide dans l'atmosphère d'un environnement intérieur ou extérieur, et plus particulièrement au contrôle du fonctionnement de tels systèmes.

Plusieurs techniques de diffusion dans l'air ambiant d'un ou plusieurs liquides existent. On en cite la diffusion par atomisation, par nébulisation, ou encore par bru-misation. En fonction des produits utilisés, cette diffusion peut avoir pour objectif de traiter l'atmosphère ambiante (odorisation/désodorisation, désinfection, ou assainissement par exemples), de soigner (aromathérapie, dermatologie), de lutter contre des nuisibles (désinsectisation, répulsion), ou d'améliorer le confort de l'espace ambiant (fraîcheur, relaxe, cosmétique, ou marketing olfactif par exemples).

Cette diffusion trouve application dans différents environnements tels que des lieux médicaux (hôpitaux, cliniques, centres thermaux), des centres d'hébergement, des hôtels, des banques, des lieux de vie, des points de vente, des centres de commerces, des centres de beauté, des lieux culturels, des centres d'affaires, ou des sites de production industrielle ou agricole.

Parmi les techniques de diffusion, celle par nébulisation préserve les propriétés du liquide à diffuser et, grâce à la transformation de ce liquide en très fines particules légères qui peuvent rester longtemps en suspension dans l'atmosphère, est relativement efficace.

Associées à ces avantages, des solutions d'automatisation et d'asservissement du fonctionnement des nébuliseurs ont aussi été proposées. Le temps de nébulisation et/ou l'intensité de cette nébulisation peuvent, en effet, être programmés et automatiquement ajustés en fonction de paramètres relatifs à l'environnement de diffusion tel que la température, la détection d'une odeur prédéfinie, ou le taux d'occupation de cet environnement. Le document US 2019/061466 décrit un système pour améliorer la qualité de l'air à l'intérieur d'un véhicule.

Néanmoins, ces solutions de l'art antérieur sont imparfaites, notamment par rapport à la pertinence des paramètres pris en considération pour contrôler/adapter le fonctionnement des nébuliseurs. Une commande inadaptée d'un dispositif de diffusion peut causer une surconsommation du liquide nébulisé, une surconcentration de ce liquide dans l'atmosphère ou dans des zones particulières de celui-ci, ainsi que toutes les conséquences qui peuvent en résulter pour les occupants ou, au contraire, un rendement insuffisant ne permettant pas d'atteindre un résultat souhaité.

Un objet de la présente invention est de remédier aux inconvénients précités.

Un autre objet de la présente invention est d'améliorer le contrôle temps-réel des nébuliseurs en fonction de paramètres relatifs à l'environnement qui sont variables et, éventuellement, non prévues à l'avance par un programme prédéfini de diffusion.

Un autre objet de la présente invention est d'affiner la commande et améliorer les performances d'asservissement des dispositifs de diffusion à l'effet de répondre au mieux aux besoins des utilisateurs.

Un autre objet de la présente invention est d'assurer une diffusion par nébulisation adaptée au contexte actuel, voire instantané, dans l'espace en question.

Un autre objet de la présente invention est d'assurer, dans un endroit spécifique, une répartition contrôlée (uniforme, multiforme, homogène ou hétérogène par exemples) d'un flux gazeux nébulisé.

Un autre objet de la présente invention est d'optimiser le rendement des nébuliseurs ou, plus généralement, des dispositifs de diffusion.

Un autre objet de la présente invention est d'améliorer l'expérience utilisateur dans un environnement de diffusion.

Un autre objet de la présente invention est de permettre une configuration simultanée d'une pluralité de dispositifs de diffusion disposés dans un environnement, afin de produire une signature olfactive prédéfinie dynamique.

A cet effet, il est proposé un système de diffusion d'un liquide selon la revendication 1.

Diverses caractéristiques supplémentaires peuvent être prévues, seules ou en combinaison :
le capteur de qualité d'air est un capteur d'odeur, un capteur de gaz, un capteur de particules, ou un capteur de bactéries ;
ladite pluralité de capteurs comprend, en outre, un capteur d'image configuré pour capter une image qui couvre au moins partiellement l'environnement ;
l'unité de contrôle est configurée pour reconnaître un individu compris dans ladite image, l'unité de contrôle étant, en outre, configurée pour ajuster ledit paramètre de fonctionnement sur la base d'au moins l'individu reconnu ;
l'unité de contrôle est configurée pour reconnaître un état émotionnel d'un individu compris dans ladite image, l'unité de contrôle étant, en outre, configurée pour ajuster ledit paramètre de fonctionnement sur la base d'au moins l'état émotionnel reconnu ; l'unité de contrôle est configurée pour estimer un nombre d'individus compris dans ladite image, l'unité de contrôle étant, en outre, configurée pour ajuster ledit paramètre de fonctionnement sur la base d'au moins le nombre d'individus estimé ;
le capteur d'image est mobile ;
le capteur d'image est porté par un véhicule aérien sans équipage à bord ou par un véhicule électrique sur rail ;
le dispositif de diffusion comprend un ventilateur soufflant configuré pour pousser dans une direction réglable un flux gazeux produit par ledit dispositif de diffusion.

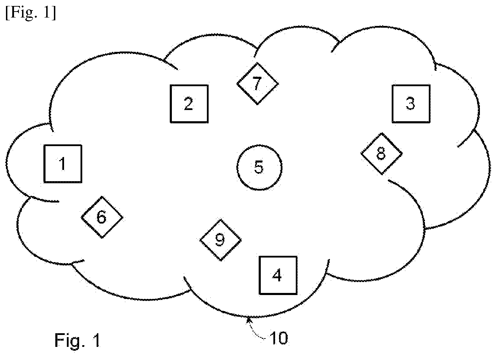

D'autres caractéristiques et avantages de l'invention apparaîtront plus clairement et de manière concrète à la lecture de la description ci-après de modes de réalisation, laquelle est faite en référence à la figure [Fig.1] qui illustre schématiquement un système de diffusion selon divers modes de réalisation.

En se référant à la figure unique, il est représenté schématiquement un système de diffusion **1-9** configuré pour diffuser un ou plusieurs liquides dans un environnement **10.**

L'environnement **10** est tout espace intérieur, semi-intérieur ou extérieur intégrant une ou plusieurs zones où au moins un liquide est à diffuser. Cet environnement **10** est, par exemples,
un point de vente à un ou plusieurs étages tel qu'une pharmacie, un magasin spécialisé, une superette, un magasin de grande surface, un supermarché, ou encore un hypermarché ;
un établissement de restauration intégrant, par exemples, une cuisine, une salle à manger, une salle fumeur, une salle non-fumeur, des cabinets d'aisances, une terrasse et/ou un local renfermant des produits alimentaires;
un complexe immobilier tel qu'un centre hospitalier, une clinique médicale, un centre thermal, un centre commercial, un centre d'affaires, un centre de déchets, un poulailler, un espace de production industrielle ou agricole, un aéroport, ou un espace touristique, culturel, de détente ou de loisirs tel qu'un hôtel, ou un cinéma;
un lieu de vie individuelle ou collective tel qu'un domicile privé, une unité de séjour, ou une maison de retraite.

On entend ici par zone tout espace de l'environnement **10** tel qu'une entrée, une salle, une chambre, une halle, un rayon, un couloir, un étage ou une partie de celui-ci.

Le système de diffusion comprend une pluralité de dispositifs de diffusion **1-4** disposés dans l'environnement **10.** Ces dispositifs de diffusion ont des paramètres de fonctionnement réglables. Le système de diffusion comprend aussi une unité de contrôle **5** configurée pour contrôler le fonctionnement des dispositifs de diffusion **1-4.** Ces dispositifs de diffusion **1-4** sont, sous le contrôle de l'unité de contrôle **5,** aptes à diffuser dans l'atmosphère, notamment par nébulisation, un ou plusieurs liquides.

Chacun des dispositifs de diffusion **1-4** est en communication fluidique avec un ou plusieurs récipients comprenant, respectivement, un liquide destiné à être diffusé dans une ou plusieurs zones de l'environnement **10.**

Un liquide destiné à être diffusé par nébulisation dans une zone de l'environnement **10,** séparément ou en combinaison avec d'autres, est, par exemples, un liquide à pouvoir odoriférant tel qu'un parfum d'intérieur ou une essence aromatique, une eau enrichie par des plantes et/ou des vitamines, une huile essentielle, une eau thermale, un neutralisateur d'odeur, un liquide médicamenteux, un désodorisant, ou une solution chimique à pouvoir désinfectant, insecticide, répulsif, bactériostatique, virucide, ou fongicide.

Plus généralement, les dispositifs de diffusion **1-4** transforment un produit, notamment des composés liquides actifs ou aromatiques, en micro particules sèches volatiles se comportant, dans l'atmosphère de l'environnement **10,** comme un gaz.

Dans un mode de réalisation, au moins un des dispositifs de diffusion **1-4** est un nébuliseur (notamment, à effet venturi). Ce nébuliseur comprend des moyens d'extraction (tel qu'un compresseur à air ou une pompe à air) et une électrovanne (ou électrovalve) réglables et contrôlables à distance par l'unité de contrôle **5.** Les moyens d'extraction sont aptes à extraire un liquide depuis un récipient à un débit et une pression donnés réglables. Le nébuliseur transforme le liquide à diffuser en un brouillard de très fines gouttelettes sous forme de flux gazeux qui peut être maintenu en suspension dans l'atmosphère de l'environnement **10.**

Le système de diffusion comprend, en outre, une pluralité de capteurs **6-9.** Ces capteurs **6-9** sont agencés pour mesurer ou capter des données destinées à être utilisées par l'unité de contrôle **5.** Ces données sont exploitées par l'unité de contrôle **5** afin d'ajuster au moins un paramètre de fonctionnement des dispositifs de diffusion **1-4.**

Ladite pluralité de capteurs **6-9** comprend au moins un capteur de qualité d'air. Ce capteur de qualité d'air permet de détecter la présence d'au moins une molécule ou d'un gaz prédéfinis dans l'atmosphère de l'environnement **10** ou d'une zone de celui-ci. Dans un autre mode de réalisation, le capteur de qualité d'air détermine la proportion d'un gaz prédéfini dans l'atmosphère environnante.

Par exemple, ce capteur de qualité d'air est un dispositif qui permet de changer ces propriétés physiques suite à une exposition à un gaz prédéfini. La transformation de ce changement en un signal électrique mesurable permet d'obtenir des informations sur la nature des gaz détectés et éventuellement leurs concentrations. Ce capteur de qualité d'air est, par exemples, un capteur à base des oxydes métalliques (MOX), un capteur à base des polymères, un capteur optique par détection infrarouge, ou un capteur à base de nanotubes de carbone.

Plus généralement, le capteur de qualité d'air vise à mesurer la qualité de l'air intérieur dans l'environnement **10.** Pour cela, au moins une particule et/ou un gaz dans l'atmosphère de l'environnement **10** sont observés par un capteur de qualité d'air. Le gaz à détecter est, par exemples, un gaz toxique tel que le dioxyde de carbone (CO2), ou le monoxyde de carbone (CO), formaldéhyde, et/ou des particules en suspension dans l'air de natures physique ou biologiques. Dans un autre mode de réalisation, le capteur de qualité d'air détecte la présence ou estime la concentration dans l'air ambiant d'un gaz prédéfini, de la substance porteuse (telle que l'eau) de la substance active diffusée dans l'environnement **10,** et/ou de cette même substance active.

A titre d'exemples non exhaustifs, le capteur de qualité d'air est un capteur d'odeur connu dans l'état de la technique par « nez électronique », un capteur de gaz, un capteur de particules, un capteur de poussières, et/ou un capteur de bactéries.

Dans certains modes de réalisation, le capteur de qualité d'air relève/capte un échantillon du gaz en continu, à une fréquence prédéfinie, ou sur demande de l'unité de contrôle **5.**

Le capteur de qualité d'air est distinct de chacun des dispositifs de diffusion **1-4.** Le capteur de qualité d'air est installé dans un emplacement différent de celui des dispositifs de diffusion **1-4.** Dans un mode de réalisation, le capteur de qualité d'air est installé sur une face en regard ou, plus généralement, différente de celle d'un dispositif de diffusion **1-4.**

Le capteur de qualité d'air comprend un ventilateur aspirant. Ce ventilateur d'air est configuré pour aspirer ou attirer dans une direction donnée de l'air vers le capteur de qualité d'air afin que ce dernier puisse mesurer la qualité de l'air aspiré dans cette direction. La direction du ventilateur aspirant équipant le capteur de qualité d'air est, dans un mode de réalisation, réglable et, de préférence, contrôlable par l'unité de contrôle **5.** Ce ventilateur aspirant peut être directionnel, bidirectionnel ou omnidirectionnel.

En outre, en étant doté d'un ventilateur aspirant, le capteur de qualité d'air peut être installé à différents endroits, y compris notamment des endroits confinés ou partiellement cloîtrés (derrière une cloison à l'abri des regards ou dans un coin d'une salle par exemples), tout en étant apte à fournir des mesures représentatives (c.à.d. fidèle à ou proche de la réalité) de la dispersion réelle d'un produit nébulisé ou, plus généralement, de la qualité de l'air.

Pour couvrir des espaces larges, plusieurs capteurs de qualité d'air, respectivement, pourvus d'un ou de plusieurs ventilateurs aspirants peuvent être envisagés. Ces capteurs de qualité d'air peuvent être disséminés dans le l'environnement **10** suivant un maillage prédéfini (par étage, par salle, par rayon, ou par zone par exemples).

En fonction des données retournées par le ou les capteurs de qualité d'air associés à une zone de l'environnement **10,** l'unité de contrôle **5** ajuste au moins un paramètre de fonctionnement d'un dispositif de diffusion **1-4** correspondant (tel que le liquide, le débit, la puissance et/ou la direction de diffusion). Il en résulte, avantageusement, qu'en fonction d'un résultat recherché (telles qu'une répartition uniforme et homogène du flux gazeux, la focalisation sur une direction donnée ou plutôt l'exclusion d'une certaine direction), l'unité de contrôle **5** adapte la commande des dispositifs de diffusion **1-4** (en agissant, notamment, sur les compresseurs, les électrovannes, l'orientation, et/ou les ventilateurs soufflant des dispositifs de diffusion **1-4**)**.**

Avantageusement, en analysant les données fournies par un capteur de qualité d'air pourvu d'un ventilateur aspirant, l'unité de contrôle **5** détermine si la qualité de l'air mesurée dans une direction/orientation donnée du ventilateur aspirant est différente d'une valeur souhaitée et ajuste, en conséquence, au moins un paramètre de fonctionnement des dispositifs de diffusion **1-4** associés au préalable à ladite direction du ventilateur aspirant. Cette direction indique, par exemples,
la source d'une odeur ou d'un gaz particulier (tabac, friture, cuisson, odeur acide, virus, champignons, bactéries, ou germe par exemples) à l'effet de focaliser la diffusion dans cette direction et permettre une neutralisation plus rapide de cette odeur;
un axe de symétrie d'une zone (celui d'un couloir ou d'un rayon par exemples), la direction d'entrée dans une zone ou la direction d'un endroit, un objet ou un individu cible (tels qu'un bureau, un stand, un sofa ou un siège par exemples) à l'effet de privilégier la diffusion dans cette direction ;
la limite d'une zone de l'environnement **10** ou d'une sous-zone à exclure (par exemple, des plantes ou des produits alimentaires sensibles au liquide nébulisé) à l'effet de restreindre la diffusion dans cette direction.

Avantageusement, des mesures effectuées par les capteurs de qualité d'air de la qualité de l'air aspiré selon des directions délimitant une zone de l'environnement **10** permet de délimiter virtuellement la diffusion du liquide dans l'atmosphère de cette zone. Ceci permet, par exemple, de localiser la diffusion d'un liquide particulier dans une zone cible de l'environnement **10** (un traitement d'air localisé) tel qu'un stand dans un salon ou un rayon dans un magasin (une odorisation par rayon).

Par ailleurs, le déploiement d'une pluralité de capteurs de qualité d'air dans l'environnement **10** permet de maintenir une signature/empreinte (olfactive, par exemple) prédéfinie de diffusion dans cet environnement **10.** Cette signature (ou empreinte) peut être définie par le flux gazeux à différentes concentrations d'un ou plusieurs liquides nébulisés et couvrir une ou plusieurs zones de l'environnement **10.** Ces capteurs de qualité d'air, équipés de ventilateurs aspirants, permettent, en effet, de détecter toute modification dans cette signature de façon à permettre à l'unité de contrôle **5** de commander le ou les dispositifs de diffusion **1-4** appropriés pour corriger cette modification. Une modification dans cette signature peut être dûe, par exemples, à la température, l'humidité, le nombre de personnes présents, un courant d'air dans l'environnement **10** ou une zone de celui-ci, ou la propagation d'un gaz. Un disfonctionnement d'un dispositif de diffusion **1-4**, l'épuisement d'un liquide en nébulisation et/ou l'apparition d'un obstacle venant s'interposer entre les capteurs de qualité d'air et les dispositifs de diffusion **1-4** peuvent être aussi à l'origine de cette modification. En conséquence, l'unité de contrôle **5** réajuste les paramètres de fonctionnement des dispositifs de diffusion **1-4** (notamment, le débit, la puissance et/ou la direction de diffusion) afin d'assurer la signature prédéfinie du flux gazeux dans l'espace de l'environnement **10.** Dans ce cas, l'unité de contrôle **5** détermine une reconfiguration pour chaque dispositif de diffusion **1-4** concerné par la zone en question de l'environnement **10** et, en temps réel, commande cette reconfiguration.

Egalement, les capteurs de qualité d'air permettent de déterminer si la puissance d'un dispositif de diffusion **1-4** convient aux dimensions de la zone à couvrir de l'environnement **10** (c.à.d. un dimensionnement).

Par ailleurs, les capteurs **6-9** équipant le système de diffusion comprennent aussi au moins un capteur de température et/ou d'humidité, permettant à l'unité de contrôle **5** de prendre en considération, dans la commande des dispositifs de diffusion **1-4**, la température ambiante et/ou l'humidité de l'air dans l'environnement **10**. La température et l'humidité de l'air dans l'atmosphère de l'environnement **10** influencent la propagation et l'aptitude des fines gouttes à être en suspension et, par conséquent, leur dispersion dans l'atmosphère de l'environnement **10**.

Les capteurs **6-9** comprennent aussi au moins un capteur d'image (ou capteur visuel). Ce capteur d'image est, par exemples, un appareil photo, une caméra numérique, une caméra 2D/3D, ou une caméra 3D à détection de profondeur permettant de capter des données d'image concernant une zone de l'environnement **10.** Ce capteur d'image permet l'acquisition d'images fixes ou vidéo intégrant au moins partiellement une zone de l'environnement **10.**

Dans un mode de réalisation, un ou plusieurs capteurs d'image sont installés dans une zone de l'environnement **10** de sorte à permettre la capture d'au moins une image d'un individu se trouvant dans cette zone. Cette capture vise la reconnaissance (visuelle) d'un individu compris dans ces images, et/ou l'estimation du nombre d'individus compris dans ces images, et/ou la reconnaissance d'un état émotionnel (c.à.d. l'humeur telle que la joie, la tristesse, la peur, le dégoût, un état neutre ou la colère par exemples) d'un individu compris dans ces images. Dans d'autres modes de réalisation, cette capture d'images vise aussi la reconnaissance du sexe et/ou la tranche d'âge d'un individu compris dans des images acquises.

En fonction des dimensions de l'environnement **10** et l'étendu du champ de vision du capteur d'image, plusieurs capteurs d'image peuvent être envisagés de sorte que le dénombrement des individus présents dans l'environnement **10** et/ou la reconnaissance d'un ou de plusieurs de ces individus et/ou l'humeur d'un ou de plusieurs de ces individus soient proches de la réalité existante. En variante ou en combinaison, au moins un des capteurs d'image est mobile. Ce capteur d'image mobile peut, en effet, être porté par un véhicule aérien sans équipage à bord (couramment appelé « drone ») ou par un véhicule électrique sur rail. Un avantage d'un capteur d'image mobile est qu'il permet de couvrir une plus grande zone de l'environnement **10.**

Dans un mode de réalisation, la reconnaissance de l'état émotionnel d'un individu présent dans une zone de l'environnement **10** est estimée par l'expression faciale (un capteur d'émotion). L'acquisition d'images successives et/ou d'images depuis plusieurs capteurs d'image permet, avantageusement, de faciliter cette reconnaissance.

Dans un autre mode de réalisation, une estimation du nombre des individus présents dans l'environnement **10** est obtenue en utilisant un capteur de mouvement configuré pour détecter le déplacement et/ou l'entrée/sortie d'un individu dans l'environnement **10** ou dans une zone de celui-ci. En variante ou en combinaison, le capteur de mouvement informe l'unité de contrôle **5** de toute détection de mouvement dans sa zone de couverture à l'effet d'ordonner la capture d'une image. Dans certains modes de réalisation, le capteur d'image et, éventuellement, un capteur de qualité d'air sont couplés à un ou plusieurs capteurs de mouvement, de sorte que la détection d'un mouvement dans une zone de l'environnement **10** déclenche automatiquement la capture d'une ou de plusieurs images couvrant cette zone et l'analyse de la qualité de l'air dans cette zone. Ainsi, l'unité de contrôle **5** peut demander au capteur d'image l'acquisition continue ou discontinue pendant un temps prédéfini corrélé à la détection de mouvement par le capteur de mouvement.

L'unité de contrôle **5** est configurée pour reconnaitre, à partir des données collectées depuis les capteurs d'image, l'identité ou l'humeur d'une personne présente dans l'environnement **10** et dont des informations distinctives ont été enregistrés au préalable. Pour cela, l'unité de contrôle **5** est pourvue d'une base de données stockant des informations relatives à un ou plusieurs individus et/ou humeurs établies au préalable. Ces informations comprennent, par exemple, un ou plusieurs modèle(s) visuel(s) pour chaque individu ou humeur. On entend par modèle visuel d'un individu toute description basée sur des descripteurs et/ou des propriétés visuelles distinctive d'un individu et/ou d'une humeur.

Ainsi, une corrélation peut être mesurée entre l'image d'un individu présent dans des images acquises et des modèles visuels préenregistrés afin d'estimer l'identité de cet individu et/ou son humeur. Cette base de données comprend, en outre, des métadonnées relatives à des configurations prédéfinies de diffusion dans l'environnement **10** associées à un ou plusieurs profils. Pour cela, l'unité de contrôle **5** comprend une application de reconnaissance d'image permettant d'extraire, sous formes d'attributs par exemples, à partir des images acquises des paramètres pertinents et de comparer ces paramètres (en utilisant l'un des divers algorithmes de reconnaissance d'image connus) aux informations, mémorisées au préalable, relatives à une liste d'individus ou d'états émotionnels. Suite à cette comparaison, l'application de reconnaissance d'image détermine, à un indice de confiance près, des informations (identité, et/ou humeur, et/ou sexe, et/ou tranche d'âge par exemples) relatives à un individu. En fonction des informations déterminées, l'unité de contrôle commande une configuration des paramètres de fonctionnement associée au préalable à ces informations.

Dans un mode de réalisation, la reconnaissance d'image par l'unité de contrôle **5** comprend, avantageusement, un modèle d'apprentissage profond (en anglais, « Deep leaming ») ou tout autre modèle basé sur une méthode d'apprentissage automatique permettant de reconnaitre, avec un indice (ou coefficient) de confiance, un individu, un état émotionnel d'un individu et/ou le nombre d'individus présents dans une ou plusieurs images acquises. Des images prises par les capteurs d'image sous diverses conditions (éclairage, occultation, angle de vue par exemples) peuvent, en effet, être utilisées pour améliorer les performances de l'application de reconnaissance d'image. Ceci permet d'améliorer la capacité à reconnaître et à identifier des individus et/ou des états émotionnels dans des conditions d'éclairages différentes et avec des images partielles ou complètes d'individus.

Dans un autre mode de réalisation, l'application de reconnaissance d'image met en oeuvre des réseaux de neurones convolutifs aptes à apprendre par eux-mêmes, à partir des données d'image acquises, des informations distinctives permettant d'estimer, à l'aide d'un apprentissage profond, une information relative à un état émotionnel ou à un individu compris dans les images acquises.

Par ailleurs, l'application de reconnaissance d'image applique, tel qu'il est connu de l'état de la technique, des algorithmes de reconnaissance faciale, des algorithmes de détection et de suivi de visages et des algorithmes de classification. Ce traitement d'image peut être réalisé en local par l'unité de contrôle **5** et/ou à distance au moyen d'un serveur de traitement distant auquel l'unité de contrôle **5** est connectée.

Par ailleurs, le système de diffusion comprend un capteur de niveau du liquide à nébuliser (un détecteur de niveau ou un capteur de poids) configuré pour mesurer le niveau du liquide dans un récipient. L'unité de contrôle **4** surveille le niveau du liquide et alerte l'opérateur dès que ce niveau est en dessous d'un seuil prédéfini.

L'unité de contrôle **5** analyse les données récoltées par des capteurs **6-9** et ajuste, en conséquence, au moins un paramètre de fonctionnement des dispositifs de diffusion **1-4.** En effet, selon les informations récupérées depuis les capteurs **6-9,** l'unité de contrôle **5** détermine une configuration appropriée des dispositifs de diffusion **1-4** permettant d'atteindre un objectif cible de nébulisation (réaliser une empreinte olfactive, traiter une substance toxique ou un air malodorant par exemples). L'unité de contrôle **5** se charge, ensuite, d'implémenter cette configuration.

Dans un mode de réalisation, la détermination par l'unité de contrôle **5** des paramètres de fonctionnement des dispositifs de diffusion **1-4** prend en considération, outre les informations produits par les capteurs **1-4** (qualité de l'air, données d'image), les paramètres de fonctionnement déterminées antérieurement. L'unité de contrôle 5 peut, en effet, utiliser l'historique des paramètres de fonctionnement des dispositifs de diffusion **1-4** pour en déterminer ceux à commander en réponse à des informations reçues depuis les capteurs **6-9,** ou pour anticiper une configuration subséquente d'un ou de plusieurs dispositifs de diffusion **1-4.**

L'unité de contrôle **5** contrôle (c.à.d. programme, commande, active/désactive ou plus généralement modifie tout paramètre de fonctionnement réglable d'un dispositif de diffusion) les dispositifs de diffusion **1-4** de façon à sélectionner, en fonction des données fournies par les capteurs **6-9** et/ou par un opérateur, le liquide à diffuser, le débit, la puissance, la fréquence et la direction de nébulisation.

Par ailleurs, l'unité de contrôle **5** comprend au moins une interface de communications sans fil ou filaire (via un réseau local par exemple) permettant sa communication, directement ou indirectement, avec les dispositifs de diffusion **1-4,** les capteurs **6-9,** un terminal utilisateur de programmation et/ou un serveur local ou distant. Ce serveur est, par exemples, un serveur de traitement, un serveur Web et/ou un serveur de notification « Push ». Une interface de communication sans fil est, par exemple, une interface Bluetooth^{®}, HomeRF^{®}, ZigBee, Infra-rouge (IR), WiFi^{®}, WiMax^{®}, ou une interface réseau universel évolué d'accès radio terrestre (tel que le 3G, 4G ou 5G par exemples).

Dans un mode de réalisation, l'unité de contrôle **5** peut être pilotée par le terminal utilisateur de programmation (tel qu'un ordinateur, une tablette, ou un Smartphone) permettant d'intervenir sur le fonctionnement des différents modules du système de diffusion **1-9** et l'implémentation d'une configuration particulière du système de diffusion définie par un opérateur.

Plus généralement, l'unité de contrôle **5** est toute entité matérielle et/ou logicielle (du type comprenant un automate programmable ou un processeur, une mémoire, des bus informatiques et divers composants électroniques servant de support pour l'exécution de programmes informatiques) capable d'acquérir des données et d'intégrer des fonctionnalités permettant la mise en oeuvre d'un procédé de traitement de données. L'unité de contrôle **5** est, dans un mode de réalisation, un module de gestion informatisé, permettant de gérer plusieurs dispositifs de diffusion **1-4** déployés dans l'environnement **10** en fonction de données récupérées depuis des capteurs **6-9** et/ou fournies par un opérateur.

Dans un mode de réalisation, l'unité de contrôle **5** notifie à un terminal utilisateur (par exemple, une application mobile installée sur un terminal mobile) des informations/alertes concernant le système de diffusion (niveau des liquides dans les récipients, performance des dispositifs de diffusion, le dispositif de diffusion le plus/ moins sollicité, les données récupérées depuis les capteurs **6-9**, confirmer un individu déterminé avec un indice de confiance en-dessous d'une valeur prédéfinie, modification/ajout d'un profil d'un individu auquel est associé une signature de nébulisation).

Le système de diffusion selon les divers modes de réalisation décrits ci-dessus est dit intelligeant dans la mesure où les paramètres de fonctionnement de chacun des diapositifs de diffusion **1-4** sont, pour assurer une signature de nébulisation prédéfinie susceptible de varier dans le temps et dans l'espace, mis à jour automatiquement (autorégulation automatique). Ainsi, il est possible de réaliser une ambiance olfactive dynamique ou une empreinte dynamique telle qu'une diffusion qui suit un individu dans son déplacement dans l'espace en question et/ou s'adapte à son état émotionnel.

Dans une mise en oeuvre illustrative, l'environnement **10** est une maison. Le système de diffusion comprend, dans ce cas, au moins un dispositif de diffusion apte à être connecté à une pluralité de récipients. Ces récipients comprennent, respectivement, un liquide destiné à être nébulisé. Ces récipients sont, par exemple, une première bouteille contenant un parfum d'ambiance, une deuxième bouteille contenant un neutralisant d'odeur, une troisième bouteille contenant une huile essentielle relaxante et une quatrième bouteille contenant une huile essentielle facilitant l'endormissement. En fonction de l'identité de l'individu et/ou son humeur et/ou son âge estimée(s) par l'unité de contrôle **5** à partir des données d'image récupérées depuis au moins un capteur d'image, l'unité de contrôle **5** commande la connexion dudit au moins un dispositif de diffusion à un des récipients précités et ajuste ses paramètres de fonctionnement (temps de nébulisation, fréquence, puissance, orientation du ventilateur soufflant par exemples) conformément à une configuration prédéfinie. En fonction des mesures effectuées par au moins un capteur de qualité d'air, l'unité de contrôle **5** réajuste au moins un paramètre de fonctionnement des dispositifs de diffusion de sorte à atteindre une empreinte prédéfinie du liquide nébulisé. Cette empreinte du liquide nébulisé varie dans l'espace de la maison (couloirs, chambres, séjour, salle d'eau, cuisine, toilettes), dans le temps (heure de la journée ou jour de la semaine par exemple) et en fonction des données fournies par les capteurs **6-9** (humeur, individus et/ou qualité de l'air par exemples).

Par exemple, si l'état émotionnel de l'individu est « ennui », l'unité de contrôle 5 commande les dispositifs de diffusion **1-4** de sorte à produire une ambiance associée au préalable à cette humeur. A cet égard, la troisième bouteille est, dans un mode de réalisation, sélectionnée et au moins un dispositif de diffusion est activé à des puissances de diffusion et dans des directions prédéfinies. Bien entendu, au vu de la géométrie de la pièce et la position à laquelle est installé le dispositif de diffusion, cette puissance peut être variable en fonction de la direction de diffusion. Les paramètres de fonctionnement du dispositif de diffusion sont réajustés en fonction des données retournées par un ou plusieurs capteurs de qualité d'air prédéfinis. La détection d'un gaz ou d'une odeur prédéfinis déclenche, par exemple, l'utilisation de la deuxième bouteille par un autre dispositif de diffusion ou en lieu et place de la troisième bouteille jusqu'à l'atténuation ou la neutralisation du gaz ou de l'odeur détecté.

Dans un autre exemple, une signature de diffusion (c.à.d. le récipient et/ou les dispositifs de diffusion avec leurs paramètres de fonctionnements respectifs) est déterminée sur la base de préférences utilisateurs renseignées au préalable à l'unité de contrôle **5**.

Avantageusement, il résulte de l'asservissement décrit ci-dessus une diffusion contrôlée conforme et ajustée aux changements dans l'environnement de diffusion (volume, qualité de l'air, signature, occupation, contexte, présence d'individus, état émotionnel).

Avantageusement, le système de diffusion contrôlée décrit ci-dessus
permet un réajustement/réglage continu et fin des dispositifs de diffusion à l'effet d'approcher au mieux une signature prédéfinie;
traite automatiquement l'atmosphère ambiante en fonction du contexte actuel dans cet environnement sans solliciter l'intervention de l'utilisateur ;
permet, au moyen des capteurs de qualité d'air, un suivi de la qualité de l'air dans l'atmosphère de l'environnement **10** ;
permet, au moyen des capteurs d'image, un suivi de l'humeur d'un individu et/ou du nombre d'individus présents dans cet environnement **10** ;
améliore l'expérience utilisateur dans l'environnement **10**;
assure une répartition contrôlée en une pluralité de zones adjacentes ou éloignées de liquides nébulisés dans l'atmosphère de l'environnement **10** ;
s'adapte à la personne et à son environnement (VIP, personne âgée, humeur, heure de la journée par exemples) ;
permet de répondre/réagir à des scénarios qui ne peuvent être prévus à l'avance par un programme de diffusion statique prédéfini,
permet, en partant d'une première disposition des dispositifs de diffusion au sein de l'environnement **10**, d'optimiser cette disposition à l'effet d'assurer de la meilleure façon une empreinte de diffusion prédéfinie.

## Revendications

1. Système de diffusion **(1-9)** d'un liquide dans un environnement **(10),** ce système comprenant
au moins un dispositif de diffusion **(1-4)** ayant un paramètre de fonctionnement réglable et configuré pour diffuser ledit liquide, le paramètre de fonctionnement réglable étant parmi le liquide à diffuser, le débit, la puissance, la fréquence, et la direction de diffusion ;
une pluralité de capteurs **(6-9)** comprenant au moins un capteur de qualité d'air,
une unité de contrôle **(5)** configurée pour récupérer une donnée captée par au moins un capteur de ladite pluralité de capteurs **(6-9)** et pour ajuster, sur la base d'au moins ladite donnée captée, ledit paramètre de fonctionnement dudit au moins un dispositif de diffusion **(1-4),**
**caractérisé en ce que**
ledit au moins un capteur de qualité d'air intègre un ventilateur aspirant configuré pour aspirer de l'air selon une direction réglable, ledit au moins un capteur de qualité d'air étant configuré pour mesurer la qualité de l'air aspiré,
l'unité de contrôle **(5)** est configurée pour ajuster ledit paramètre de fonctionnement sur la base d'au moins la qualité de l'air aspiré selon ladite direction réglable ;
le dispositif de diffusion **(1-4)** comprend un ventilateur soufflant configuré pour pousser dans une direction réglable un flux gazeux produit par ledit dispositif de diffusion **(1-4).**

2. Système selon la revendication 1, **caractérisé en ce que** le capteur de qualité d'air est un capteur d'odeur, un capteur de gaz, un capteur de particules, ou un capteur de bactéries.

3. Système selon la revendication 1 ou 2, **caractérisé en ce que** ladite pluralité de capteurs comprend, en outre, un capteur d'image configuré pour capter une image qui couvre au moins partiellement l'environnement **(10).**

4. Système selon la revendication précédente, **caractérisé en ce que** l'unité de contrôle **(5)** est configurée pour reconnaître un individu compris dans ladite image, l'unité de contrôle **(5)** étant, en outre, configurée pour ajuster ledit paramètre de fonctionnement sur la base d'au moins l'individu reconnu.

5. Système selon la revendication 3 ou 4, **caractérisé en ce que** l'unité de contrôle **(5)** est configurée pour reconnaître un état émotionnel d'un individu compris dans ladite image, l'unité de contrôle **(5)** étant, en outre, configurée pour ajuster ledit paramètre de fonctionnement sur la base d'au moins l'état émotionnel reconnu.

6. Système selon l'une quelconque des revendications 3 à 5, **caractérisé en ce que** l'unité de contrôle **(5)** est configurée pour estimer un nombre d'individus compris dans ladite image, l'unité de contrôle **(5)** étant, en outre, configurée pour ajuster ledit paramètre de fonctionnement sur la base d'au moins le nombre d'individus estimé.

7. Système selon l'une quelconque des revendications 3 à 6, **caractérisé en ce que** le capteur d'image est mobile.

8. Système selon la revendication précédente, **caractérisé en ce qu'**il comprend, en outre, un véhicule aérien sans équipage à bord ou un véhicule électrique sur rail, le capteur d'image étant porté par le véhicule aérien sans équipage à bord ou par le véhicule électrique sur rail.

## Patentansprüche

1. Verteilungssystem (1-9) für eine Flüssigkeit in einer Umgebung (10), wobei dieses System Folgendes umfasst:
Mindestens eine Verteilungsvorrichtung (1-4), die einen einstellbaren Betriebsparameter aufweist und dazu konfiguriert ist, die Flüssigkeit zu verteilen,
wobei der einstellbare Betriebsparameter eines von der zu verteilenden Flüssigkeit, der Durchflussrate, der Leistung, der Frequenz und der Verteilungsrichtung ist;
eine Vielzahl von Sensoren (6-9), die mindestens einen Luftqualitätssensor umfassen, eine Steuereinheit (5), die dazu konfiguriert ist, Daten abzurufen, die von mindestens einem Sensor der Vielzahl von Sensoren (6-9) erfasst werden, und den Betriebsparameter der mindestens einen Verteilungsvorrichtung (1-4) basierend auf mindestens den erfassten Daten anzupassen,
**dadurch gekennzeichnet, dass**
der mindestens eine Luftqualitätssensor ein Sauggebläse umfasst, das dazu konfiguriert ist, Luft in einer einstellbaren Richtung anzusaugen, wobei der mindestens eine Luftqualitätssensor dazu konfiguriert ist, die Qualität der angesaugten Luft zu messen,
die Steuereinheit (5) dazu konfiguriert ist, den Betriebsparameter basierend auf mindestens der Qualität der angesaugten Luft in der einstellbaren Richtung anzupassen;
die Verteilungsvorrichtung (1-4) ein Gebläse umfasst, das dazu konfiguriert ist, einen von der Verteilungsvorrichtung (1-4) erzeugten Gasstrom in eine einstellbare Richtung zu drücken.

2. System nach Anspruch 1, **dadurch gekennzeichnet, dass** der Luftqualitätssensor ein Geruchssensor, ein Gassensor, ein Partikelsensor oder ein Bakteriensensor ist.

3. System nach Anspruch 1 oder 2, **dadurch gekennzeichnet, dass** die Vielzahl von Sensoren ferner einen Bildsensor umfasst, der dazu konfiguriert ist, ein Bild aufzunehmen, das mindestens teilweise die Umgebung (10) abdeckt.

4. System nach dem vorhergehenden Anspruch, **dadurch gekennzeichnet, dass** die Steuereinheit (5) dazu konfiguriert ist, eine in dem Bild enthaltene Person zu erkennen, wobei die Steuereinheit (5) ferner dazu konfiguriert ist, den Betriebsparameter basierend auf mindestens der erkannten Person anzupassen.

5. System nach Anspruch 3 oder 4, **dadurch gekennzeichnet, dass** die Steuereinheit (5) dazu konfiguriert ist, einen emotionalen Zustand einer in dem Bild enthaltenen Person zu erkennen, wobei die Steuereinheit (5) ferner dazu konfiguriert ist, den Betriebsparameter basierend auf mindestens dem erkannten emotionalen Zustand anzupassen.

6. System nach einem der Ansprüche 3 bis 5, **dadurch gekennzeichnet, dass** die Steuereinheit (5) dazu konfiguriert ist, die Anzahl der in dem Bild enthaltenen Personen zu schätzen, wobei die Steuereinheit (5) ferner dazu konfiguriert ist, den Betriebsparameter basierend auf mindestens der geschätzten Personenanzahl anzupassen.

7. System nach einem der Ansprüche 3 bis 6, **dadurch gekennzeichnet, dass** der Bildsensor beweglich ist.

8. System nach dem vorhergehenden Anspruch, **dadurch gekennzeichnet, dass** es ferner ein unbemanntes Luftfahrzeug oder ein elektrisches Schienenfahrzeug umfasst, wobei der Bildsensor von dem unbemannten Luftfahrzeug oder dem elektrischen Schienenfahrzeug getragen wird.

## Claims

1. A system (1-9) for dispensing a liquid in an environment (10), this system comprising
- at least one dispensing device (1-4) having a settable operating parameter and configured to dispense said liquid, the settable operating parameter being among the liquid to be dispensed, the flow rate, the power, the frequency and the dispensing direction;
- a plurality of sensors (6-9) comprising at least one air quality sensor,
- a control unit (5) configured to gather a datum captured by at least one sensor of said plurality of sensors (6-9) and to adjust, based on at least said captured datum, said operating parameter of said at least one dispensing device (1-4);
**characterized in that**
- said at least one air quality sensor incorporates a suction fan configured to suck air according to a settable direction, said at least one air quality sensor being configured to measure the quality of the sucked air,
- the control unit (5) is configured to adjust said operating parameter based on at least the quality of the air sucked according to said settable direction,
- the dispensing device (1-4) comprises a blower fan configured to push in a settable direction a gas flow produced by said dispensing device (1-4) .

2. The system according to claim 1, **characterized in that** the air quality sensor is an odor sensor, a gas sensor, a particle sensor, or a bacteria sensor.

3. The system according to claim 1 or 2, **characterized in that** said plurality of sensors furthers comprises an image sensor configured to capture an image which at least partially covers the environment (10).

4. The system according to the preceding claim, **characterized in that** the control unit (5) is configured to recognize an individual comprised in said image, the control unit (5) being further configured to adjust said operating parameter based on at least the recognized individual.

5. The system according to claim 3 or 4, **characterized in that** the control unit (5) is configured to recognize an emotional state of an individual comprised in said image, the control unit (5) being further configured to adjust said operating parameter based on at least the recognized emotional state.

6. The system according to any of claims 3 to 5, **characterized in that** the control unit (5) is configured to estimate a number of individuals comprised in said image, the control unit (5) being further configured to adjust said operating parameter based on at least the estimated number of individuals.

7. The system according to any of claims 3 to 6, **characterized in that** the image sensor is movable.

8. The system according to the preceding claim, **characterized in that** it further comprises an unmanned aerial vehicle or a electric rail vehicle, the image sensor being carried by the unmanned aerial vehicle or by the electric rail vehicle.
